Europäisches Patentamt
European Patent Office
Office européen des brevets

(19) (11) Publication number: **0 347 531 A2**

# EUROPEAN PATENT APPLICATION

(12)

(21) Application number: 89105293.8

(22) Date of filing: 23.03.89

(51) Int. Cl.4: **A61K 31/385**

The title of the invention has been amended (Guidelines for Examination in the EPO, A-III, 7.3).

(30) Priority: 13.09.88 JP 227492/88
20.05.88 US 196431

(43) Date of publication of application:
27.12.89 Bulletin 89/52

(84) Designated Contracting States:
BE CH DE ES FR GB IT LI NL SE

(71) Applicant: SS PHARMACEUTICAL CO., LTD.
2-12-4 Nihonbashi Hama-cho Chuo-ku
Tokyo(JP)

(72) Inventor: Nakazawa, Hitoshi
3-5-2-210, Hashigadai
Narita-shi Chiba-ken(JP)
Inventor: Ohtsuka, Mari
4-7-15-704, Yatsu
Narashino-shi Chiba-ken(JP)
Inventor: Matsuda, Hideaki
2-29-8, Higashiabiko
Abiko-shi Chiba-ken(JP)
Inventor: Katori, Tatsuhiko
3081-11, Fukawa Tone-machi
Kitasouma-gun Ibaraki-ken(JP)
Inventor: Irinoda, Kazuhiko
5-17-1-103, Masago
Chiba-ken Chiba-shi(JP)

(74) Representative: Wächtershäuser, Günter, Dr.
Tal 29
D-8000 München 2(DE)

## (54) Use of 1,3-dithiole-2-thione-derivatives in therapy of hepatic diseases.

(57) A therapeutic agent for hepatic diseases comprising a 1,3-dithiole-2-thione derivative is disclosed. The agent may be formed into tablets, granules, powders, capsules, suspensions, injections, and suppositories. Normal dose is from 0.1 to 100 mmg/kg/day for oral administration, and 0.02 to 20 mg/kg/day for injection or other forms of administration. The compounds decrease GOT and GPT values in serum dose dependently and are safer than Malotilate which is a known hepatic desease medicine.

EP 0 347 531 A2

# THERAPEUTIC AGENT FOR HEPATIC DISEASES

## BACKGROUND OF THE INVENTION

### Field of the Invention:

This invention relates to a therapeutic agent for hepatic diseases.

### Description of the Background:

Very few therapeutic agents have been known in the art which are effective for serious hepatic diseases such as cirrhosis of the liver or the like. Only diisopropyl-1,3-dithiol-2-ylidenemalonate (generally known as Malotilate), which has an amelioration effect of liver protein metabolism, is known as a medicine effective against cirrhosis of the liver.

However, since Malotilate is known to have side effects, there has been a need for the development of a compound which is safer than Malotilate and has the same or better pharmaceutical effect. Various kinds of 1,3-dithiole derivatives have been synthesized with the intention of providing such a compound. However, none of them are yet satisfactory in terms of their pharmaceutical effect and safety. Therefore, there still exists a strong need for a compound which both gives the pharmaceutical effect and is safe.

In view of this situation, the present inventors have conducted extensive screenings on a number of compounds with respect to their therapeutic effect on hepatic diseases and their safety and as result have found that certain cyclic sulfur-containing compounds are excellent therapeutic agents for hepatic diseases and can satisfy the requirements of therapeutic effectiveness and safety. Such a finding has led to the completion of this invention.

## SUMMARY OF THE INVENTION

Accordingly an object of this invention is to provide a therapeutic agent for the hepatic diseases such as cirrhosis, alcoholic hepatitis, virus-derived hepatitis, and the like, which comprises administrating to a patient an effective amount of a cyclic sulfur-containing compound represented by the following formula (I):

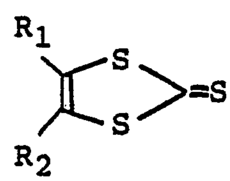

(I)

wherein the groups $R_1$ and $R_2$ are independently selected from the group consisting of hydrogen; $C_{1-20}$ alkyl groups which are unsubstituted or substituted with halogen, hydroxyl, lower alkoxy, amino, lower alkylamino, carboxyl, lower alkoxycarbonyl, or cyano; $C_{5-7}$ cycloalkyl groups; $C_{7-14}$ aralkyl groups which are unsubstituted or substituted with halogen, hydroxyl, lower alkoxy, amino, lower alkylamino, carboxyl, lower alkoxycarbonyl, or cyano; $C_{2-12}$ alkenyl groups which are unsubstituted or substituted with halogen, hydroxyl, carboxyl, or $C_{6-10}$ aryl; and $C_{6-10}$ aryl groups which are unsubstituted or substituted with halogen, lower alkyl, halo-lower alkyl, hydroxyl, lower alkoxy, lower alkylenedioxy, amino, lower alkylamino, carboxyl, lower alkoxycarbonyl, cyano, nitro, $C_{6-10}$ aryl, $C_{6-10}$ aryloxy, sulfonyl, formyl, carbamoyl, $C_{1-6}$ acyl, or $C_{7-11}$ aroyl, provided that $R_1$ and $R_2$ are not hydrogen atoms at the same time.

Other objects, features and advantages of the invention will hereinafter become more readily apparent from the following description.

## DETAILED DESCRIPTION OF THE INVENTION AND PREFERRED EMBODIMENTS

Given as specific examples of groups in the above definition of $R_1$ and $R_2$ of formula (I) are: methyl, ethyl, isobutyl, dodecyl, and the like for $C_{1-20}$ alkyl group; phenyl, naphthyl, and the like for $C_{6-10}$ aryl group; vinyl, allyl, isobutenyl, decenyl, and the like for $C_{2-12}$ alkenyl group; phenoxy, naphthoxy, and the like for $C_{6-10}$ aryloxy group; acetyl group and the like for $C_{1-6}$ acyl group; and benzoyl, naphthoyl, and the like for $C_{7-11}$ aroyl group. Given as examples of halogen atoms which may be contained in groups $R_1$ or $R_2$ are fluorine, bromine, chlorine, iodine, and the like. In the above definition and throughout this specification, a lower alkyl group means an alkyl group having 1-4 carbon atoms.

Preferred compounds of formula (I) are those having groups, as $R_1$, $C_{1-4}$ alkyl group; phenyl group which is unsubstituted or substituted with halogen, $C_{1-4}$ alkoxy, or trifluoromethyl; or phenylalkyl group which is unsubstituted or substituted with halogen; and, as $R_2$, hydrogen atom or $C_{1-4}$ alkyl group.

The compound of formula (I) can be prepared, for example, by the method proposed by J. P. Ferraris et al [Tetrahedron Letters, 2553 (1973)].

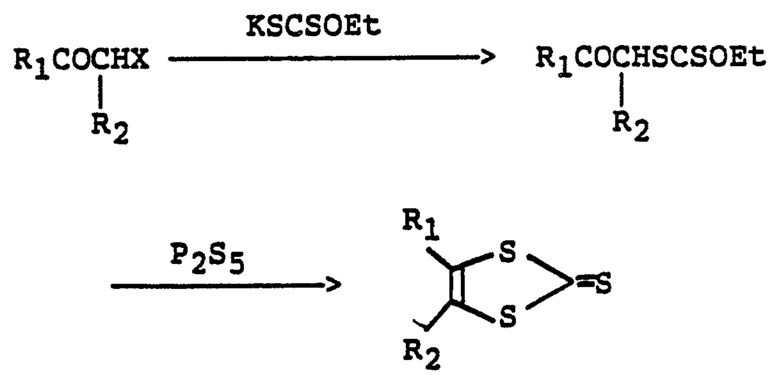


wherein X represents a halogen atom, and $R_1$ and $R_2$ have the same meanings as defined above.

The following compounds are given as specific examples of the compounds of formula (I):

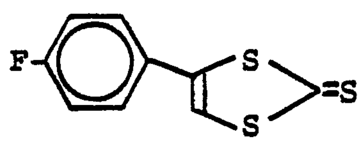

mp. 133-134°C

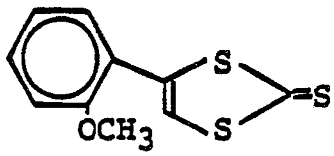

mp. 100-101°C

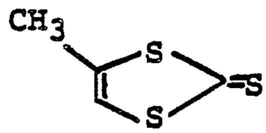

mp. 38-40°C

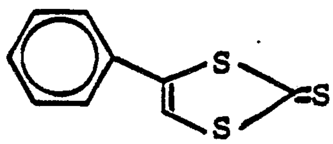

mp. 120-121°C

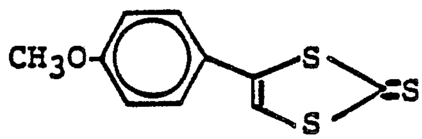

mp. 108-110°C

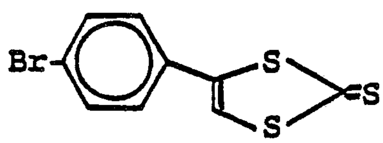

mp. 152-154°C

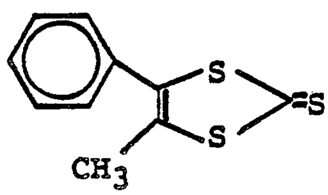

mp. 64-65°C

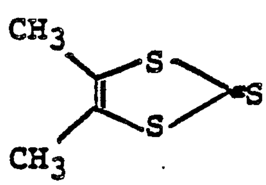

mp. 86-91°C

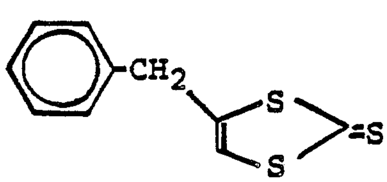

mp. 65-66°C

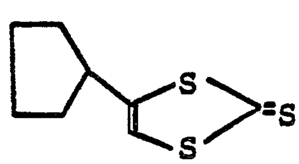

mp. 57-59°C

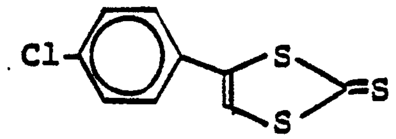

mp. 133-135°C

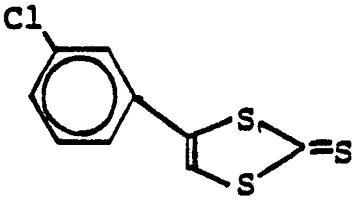

mp. 106-107°C

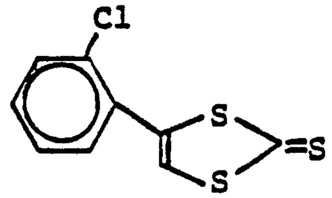

mp. 132-134°C

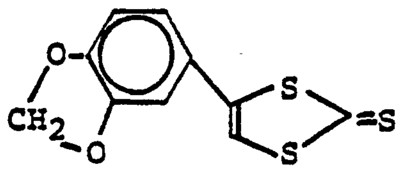

mp. 159-160°C

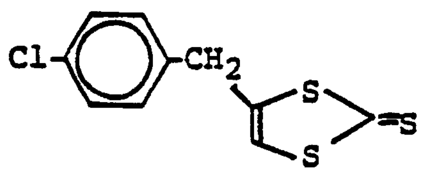

mp. 99-100°C

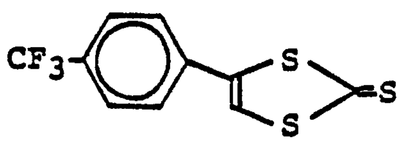

mp. 111-112°C

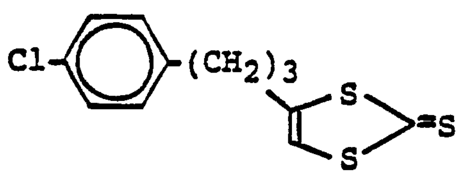

mp. 110-112°C

Up to the present, there have been no knowledge about medicinal action, particularly about hepatic disease curing or alleviating effects, of the above compounds. The first mentioned compound, 4-(4-fluorophenyl)-1,3-dithiole-2-thione [Compound (4) in Table 1], has not heretofore been reported and is a novel compound.

As shown in the tests described below, in which hepatic disorder was experimentally induced in animals by the administration of carbon tetrachloride, or carbon tetrachloride and galactosamine, the compound (I) depressed changes in the outward appearance of the liver and exhibited a remarkable suppression of GOT and GPT activities in serum.

<Experiment I>

Several groups of ddY male mice, each group consisting of 5 mice, aged 5 weeks and weighing approximately 25 g, were provided for the test. The mice were fasted for 16 hours and were given 0.05 ml/kg of carbon tetrachloride mixed with olive oil by oral administration to provide a dose (as a mixture of carbon. tetrachloride and olive oil) of 10 ml/kg. At the same time, the compounds of the present invention or Malotilate (comparative agent), each dissolved in olive oil to give the prescribed concentration, were given to each mouse in a given group. Blood was collected from each mouse at 24 hours after administration for measurement of GOT and GPT activities in serum. The results are shown in Table 1 below.

**TABLE 1**

| Compound | Dose (mg/kg p.o.) | GOT (IU/L) | GPT (IU/L) |
|---|---|---|---|
| Control Group | - | 80 | 30 |
| Groups given only carbon tetrachloride | - | 12700 | 6340 |
| (1) 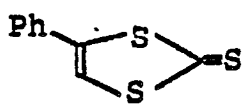 | 20<br>100 | 130<br>90 | 50<br>20 |
| (2) 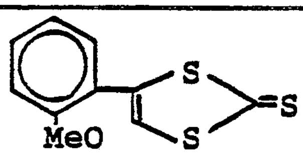 | 100 | 280 | 120 |
| (3) 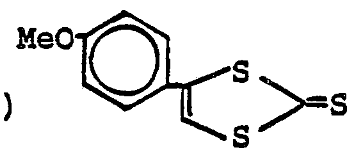 | 100 | 210 | 180 |
| (4) 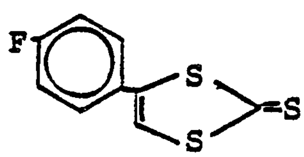 | 20<br>100 | 320<br>120 | 270<br>20 |
| (5) 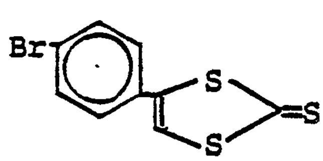 | 100 | 130 | 30 |
| (6) 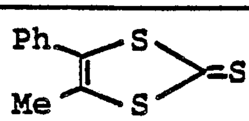 | 100 | 190 | 50 |
| Malotilate | 20<br>100<br>500 | 8540<br>3220<br>440 | 6260<br>2290<br>310 |

Histological observations were made on the test animals to detect the presence of a liver necrosis. As a

result, a centrilobular liver necrosis was found in animals of the group of which only carbon tetrachloride had been administered, while in the groups to which 100 mg/kg or more of Malotilate were given, the degree of centrilobular liver necrosis was less significant than in the group of animals to which only carbon tetrachloride had been adminstered. On the other hand, there was no centrilobular liver necrosis observed in animals of the groups to which 20-100 mg/kg or more of the compound (I) was administered.

As is evident from Table 1, the administration of carbon tetrachloride brings about a significant increase in values of serum GOT and GPT. The compounds of formula (I) and Malotilate decrease these values almost dose dependently. Such meaningful decreases can be realized with respect to compounds of formula (I), especially Compounds (1) and (4) in the table, at a dose of about 20 mg/kg or more, and with respect to Malotilate a dose of at least 500 mg/kg or more. The $LD_{50}$ values of the compounds of formula (I) were from 500 to 1,000 mg/kg (mouse; p.o.) and that of Malotilate was 4,000-8,000 mg/kg (mouse; p.o.). This evidence suggests that the compounds of formula (I) are safer than Malotilate.

<Experiment II>

Five (5) groups of Wister male rats, each group consisting of 8 rats, age 7 weeks, were provided for the test. These groups were designated as (i) Control Group I (no compound was administered to this group), (ii) Control Group II (carbon tetrachloride and D-galactosamine were administered), (iii) Compound (1) Group, (Compound (1) in Table 1 was administered), (iv) Compound (4) Group, (Compound (4) in Table 1 was administered), and (v) DT Group (1,3-dithiole-2-thione was administered). To each rat of groups (ii)-(v), 400 mg/kg of D-galactosamine was intraperitoneally and 0.1 ml/kg of carbon tetrachloride was subcutaneousely administered during 4 weeks, three times a week, provided that to the rats weighing more than 250 g 100 mg/rat of D-galactosamine and 0.025 ml/rat of carbon tetrachloride were administered at a time. To the rats of groups (iii)-(v), 10 mg/kg of Compound (1), Compound (4), or 1,3-dithiole-2-thione, each dissolved into olive oil, was orally administered every day for 4 weeks. After the end of the test period, blood was collected from each rat and GOT, GPT, and ALP activities in serum were measured. The results are shown in Table 2.

**TABLE 2**

| Group | GOT (K.U.) | GPT (K.U.) | ALP (K-AU) |
|---|---|---|---|
| Control Group I | 100.4 | 34.6 | 29.6 |
| Control Group II | 843.6 | 232.4 | 59.8 |
| Compound (1) Group | 166.5 | 51.4 | 46.0 |
| Compound (4) Group | 150.9 | 40.0 | 43.3 |
| DT Group | 254.9 | 87.1 | 53.5 |

Compound (1) and Compound (4) Groups demonstrated a remarkable decrease in GOT and GPT (1/4-1/5 of Control Group II), while the decrease for DT Group was less significant. ALP values also showed some decrease in Compound (1) and Compound (4) Groups. In contrast, 1,3-dithiole-2-thione exhibited no ALP activity suppressing effect.

As illustrated above, the compounds (I) exhibit a remarkably high liver disorder suppressing effect and safety as compared with the comparative agent Malotilate.

Since a mouse and rat are well established model animal widely employed in the medical and pharmacological fields, it is apparent that the excellent results obtained from the compounds of this invention are also applicable to humans.

The compounds of the present invention are useful in treating various acute or chronic liver disorders including alcoholic hepatitis and virus-derived hepatitis, as well as liver cirrhosis.

Although the amount of dosage of the compound (I) used as a hepatic disease therapeutic agent may vary depending on the body weight, age, sex of the subject, the manner by which it is administered, the conditions and significance of disease of the subject, and the like, a generally appropriate amount may be from 0.1 to 100 mg per day per kg of body weight of the subject when it is orally administered, and from 0.02 to 20 mg per day per kg of body weight when it is otherwise administered.

The hepatic disease therapeutic agent of the present invention can be prepared in various dosing forms according to conventional methods, such as tablets, granules, powders, capsules, suspensions, injections, suppositories, or the like. When it is prepared in a solid dosing form for oral administration, various additives such as a binder, disintegrator, glossing agent, coloring agent, flavor, extending agent, coating agent, sugar coating agent, and the like, are compounded as appropriate, and then formed into tablets, granules, powders, capsules, or the like according to conventional methods. When an injection is prepared using this compound (I) as a main active agent, the compound is dissolved as required in an oil or fat such as olive oil, sesame oil, or the like, and made into a liquid for subcutaneous muscle injection. Alternatively, it is possible to prepare an intravenous injection liquid or an infusion fluid by adding a surface active agent to the formulation. A suppository can be prepared by adding coconut butter or a medium-chain fatty acid glycerol ester to the compound (I), and by heating and kneading the mixture before shaping it into a suppository.

Other features of the invention will become apparent in the course of the following description of the exemplary embodiments which are given for illustration of the invention and are not intended to be limiting thereof.

## EXAMPLES

### Synthetic Example

Synthesis of 4-(4-fluorophenyl)-1,3-dithiole-2-thione

(i) 4.34 g of 4-fluorophenacyl bromide and 3.54 g of potassium xanthogenate was dissolved into 50 ml of ethanol and the mixture was stirred for 3 hours under ice-cooling. Ethanol was evaporated from the reaction mixture. After the ddition of ice water, the residue was extracted with ether. The ether layer was washed with water and dried over magnesium sulfate anhydride. The solvent was evaporated under reduced pressure. The residue was purified over silica gel column chromatography to obtain 2.58 g of O-ethyl-S-(4- fluorophenacyl)dithiocarbonate as yellow oily substance at a yield of 50%.
$^1$H-NMR $\delta$ ppm (in $CDCl_3$): 1.40 (t,3H), 4.66 (s, 2H), 4.68 (q, 2H), 7.05-7.45 (m, 2H), 7.90-8.30 (m, 2H)

(ii) 2.42 g of
O-ethyl-S-(4-fluorophenyl)dithio-carbonate and 6.30 g of phosphorus pentasulfide was suspended into 50 ml of decalin. The mixture was heated under reflux for 30 minutes. After cooling, 100 ml of ether was added to separate ether soluble layer. This layer was washed with water, 10% sodium hydroxide and water, and dried over magnesium sulfate anhydride. The solvent was evaporated under reduced pressure. The residue was purified over silica gel column chromatography to obtain 0.82 g of the title compound at a yield of 38%.
mp: 133-134 °C; yellow needle-like crystals
$^1$H-NMR $\delta$ ppm (in $CDCl_3$): 6.80-7.70 (m, 4H), 7.05 (s, 1H)

### Example 1 Tablet

| | |
|---|---|
| 4-phenyl-1,3-dithiole-2-thione | 50 mg |
| crystallized cellulose | 50 mg |
| lactose | 50 mg |
| hydroxypropylcellulose | 18 mg |
| magnesium stearate | 2 mg |
| Total | 170 mg |

The tablet of the above composition can be made into a sugar coated or film coated tablet.

Example 2 Capsule

| | |
|---|---|
| 4-phenyl-1,3-dithiole-2-thione | 50 mg |
| light silicic acid anhydride | 25 mg |
| lactose | 100 mg |
| starch | 50 mg |
| talc | 25 mg |
| Total | 250 mg |

The above components were filled in a No. 1 capsule.

Example 3 Granule

| | |
|---|---|
| 4-phenyl-1,3-dithiole-2-thione | 50 mg |
| lactose | 600 mg |
| corn starch | 200 mg |
| sodium carboxymethylcellulose | 20 mg |
| hydroxypropylcellulose | 130 mg |
| Total | 1,000 mg |

The above amounts of the components were made into a granule by a conventional method.

Example 4 Powder

| | |
|---|---|
| 4-phenyl-1,3-dithiole-2-thione | 50 mg |
| light silicic acid anhydride | 20 mg |
| precipitating calcium carbonate | 10 mg |
| lactose | 250 mg |
| starch | 70 mg |
| Total | 400 mg |

The above amounts of the components were made into a powder by a conventional method.

Example 5 Suppository

| | |
|---|---|
| 4-phenyl-1,3-dithiole-2-thione | 10 mg |
| coconut butter | 890 mg |
| Total | 900 mg |

The above amounts of the components were made into a suppository by a conventional method.

Example 6 Injection

| | |
|---|---|
| 4-phenyl-1,3-dithiole-2-thione | 10 mg |
| hydrogenated castor oil | 80 mg |
| propylene glycol | 60 mg |
| dextrose | 50 mg |

The above components were dissolved in distilled water for injection to make the total volume to 1 ml to prepare an injection fluid by a conventional method.

Example 7 Tablet

| | |
|---|---|
| 4-phenyl-1,3-dithiole-2-thione | 2 mg |
| crystallized cellulose | 74 mg |
| lactose | 74 mg |
| hydroxypropylcellulose | 18 mg |
| magnesium stearate | 2 mg |
| Total | 170 mg |

The tablet of the above composition can be made into a sugar coated or film coated tablet.

Example 8 Injection

| | |
|---|---|
| 4-phenyl-1,3-dithiole-2-thione | 1 mg |
| polyoxyethylene hydrogenated castor oil | 40 mg |
| propylene glycol | 60 mg |

The above components were dissolved in distilled water for injection to make the total volume to 1 ml to prepare an injection fluid by a conventional method.

Example 9 Tablet

| | |
|---|---|
| 4-(4-fluorophenyl)-1,3-dithiole-2-thione | 50 mg |
| crystallized cellulose | 50 mg |
| lactose | 50 mg |
| hydroxypropylcellulose | 18 mg |
| magnesium stearate | 2 mg |
| Total | 170 mg |

The tablet of the above composition can be made into a sugar coated or film coated tablet.

Example 10 Capsule

| | |
|---|---|
| 4-(4-fluorophenyl)-1,3-dithiole-2-thione | 50 mg |
| light silicic acid anhydride | 25 mg |
| lactose | 100 mg |
| starch | 50 mg |
| talc | 25 mg |
| Total | 250 mg |

The above components were filled in a No. 1 capsule.

Example 11 Granule

| | |
|---|---|
| 4-(4-fluorophenyl)-1,3-dithiole-2-thione | 50 mg |
| lactose | 600 mg |
| corn starch | 200 mg |
| sodium carboxymethylcellulose | 20 mg |
| hydroxypropylcellulose | 130 mg |
| Total | 1,000 mg |

The above amounts of the components were made into a granule by a conventional method.

Example 12 Powder

| | |
|---|---|
| 4-(4-fluorophenyl)-1,3-dithiole-2-thione | 50 mg |
| light silicic acid anhydride | 20 mg |
| precipitating calcium carbonate | 10 mg |
| lactose | 250 mg |
| starch | 70 mg |
| Total | 400 mg |

The above amounts of the components were made into a powder by a conventional method.

Example 13 Suppository

| | |
|---|---|
| 4-(4-fluorophenyl)-1,3-dithiole-2-thione | 10 mg |
| coconut butter | 890 mg |
| Total | 900 mg |

The above amounts of the components were made into a suppository by a conventional method.

Example 14 Injection

| | |
|---|---|
| 4-(4-fluorophenyl)-1,3-dithiole-2-thione | 10 mg |
| hydrogenated castor oil | 80 mg |
| propylene glycol | 60 mg |
| dextrose | 50 mg |

The above components were dissolved in distilled water for injection to make the total volume to 1 ml to prepare an injection fluid by a conventional method.

Example 15 Tablet

| | |
|---|---|
| 4-(4-fluorophenyl)-1,3-dithiole-2-thione | 2 mg |
| crystallized cellulose | 74 mg |
| lactose | 74 mg |
| hydroxypropylcellulose | 18 mg |
| magnesium stearate | 2 mg |
| Total | 170 mg |

The tablet of the above composition can be made into a sugar coated or film coated tablet.

Example 16 Injection

| | |
|---|---|
| 4-(4-fluorophenyl)1,3-dithiole-2-thione | 1 mg |
| polyoxyethylene hydrogenated castor oil | 40 mg |
| propylene glycol | 60 mg |

The above components were dissolved in distilled water for injection to make the total volume to 1 ml to prepare an injection fluid by a conventional method.

Obviously, numerous modifications and variations of the present invention are possible in light of the above teachings. It is therefore to be understood that the scope of the appended claims, the invention may be practiced otherwise than as specifically described herein.

## Claims

1. A therapeutic agent for hepatic diseases characterised by comprising a cyclic sulfur-containing compound represented by the following formula (I),

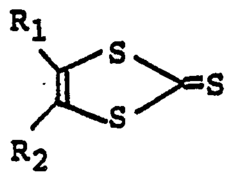

(I)

wherein the groups $R_1$ and $R_2$ are independently selected from the group consisting of hydrogen; $C_{1-20}$ alkyl groups which are unsubstituted or substituted with halogen, hydroxyl, lower alkoxy, amino, lower alkylamino, carboxyl, lower alkoxycarbonyl, or cyano; $C_{5-7}$ cycloalkyl groups; $C_{7-14}$ aralkyl groups which are unsubstituted or substituted with halogen, hydroxyl, lower alkoxy, amino, lower alkylamino, carboxyl, lower alkoxycarbonyl, or cyano; $C_{2-12}$ alkenyl groups which are unsubstituted or substituted with halogen, hydroxyl, carboxyl, or $C_{6-10}$ aryl; and $C_{6-10}$ aryl groups which are unsubstituted or substituted with lower alkyl, hydroxyl, lower alkoxy, lower alkylenedioxy, amino, lower alkylamino, carboxyl, lower alkoxycarbonyl, cyano, nitro, $C_{6-10}$ aryl, $C_{6-10}$ aryloxy, sulfonyl, formyl, carbamoyl, $C_{1-6}$ acyl, or $C_{7-11}$ aroyl, provided that $R_1$ and $R_2$ are not hydrogen atoms at the same time.

2. The therapeutic agent for hepatic diseases according to Claim 1, wherein $R_1$ is selected from the group consisting of $C_{1-4}$ alkyl group; phenyl group which is unsubstituted or substituted with $C_{1-4}$ alkoxy; and phenylalkyl group which is unsubstituted or substituted with halogen; and $R_2$ is a hydrogen atom or a $C_{1-4}$ alkyl group.

3. The therapeutic agent for hepatic diseases according to Claim 1, wherein the hepatic disease is cirrhosis, alcoholic hepatitis, or virus-derived hepatitis.

4. The therapeutic agent for hepatic diseases according to Claim 1 further comprising a pharmacologically acceptable carrier.